(19) **Europäisches Patentamt / European Patent Office / Office européen des brevets**

(11) **EP 2 961 454 B1**

(12) **EUROPEAN PATENT SPECIFICATION**

(45) Date of publication and mention of the grant of the patent:
**08.06.2022 Bulletin 2022/23**

(21) Application number: **14757421.4**

(22) Date of filing: **28.02.2014**

(51) International Patent Classification (IPC):
*A61M 11/00* (2006.01)  *A61L 9/14* (2006.01)
*B05B 17/06* (2006.01)  *B06B 1/06* (2006.01)
*A61M 15/00* (2006.01)  *B05B 17/00* (2006.01)

(52) Cooperative Patent Classification (CPC):
**A61L 9/14; A61M 11/005; A61M 15/0085;
B05B 17/0607; B05B 17/0615; B05B 17/0676;
B05B 17/0684; B06B 1/0644;** A61L 2209/132

(86) International application number:
**PCT/IB2014/059321**

(87) International publication number:
**WO 2014/132228 (04.09.2014 Gazette 2014/36)**

(54) **ATOMISATION APPARATUS USING SURFACE ACOUSTIC WAVE GENERATION**

ATOMISIERUNGSVORRICHTUNG MIT ERZEUGUNG VON OBERFLÄCHENKLANGWELLEN

APPAREIL D'ATOMISATION UTILISANT LA GÉNÉRATION D'ONDE ACOUSTIQUE DE SURFACE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **01.03.2013 AU 2013900690**

(43) Date of publication of application:
**06.01.2016 Bulletin 2016/01**

(73) Proprietor: **Rmit University
Melbourne, Victoria 3000 (AU)**

(72) Inventors:
• **FRIEND, James
Oakleigh East, Victoria 3166 (AU)**
• **YEO, Leslie
Malvern, Victoria 3144 (AU)**
• **QI, Aisha
Clayton South, Victoria 3169 (AU)**

(74) Representative: **Aulich, Martin et al
Meissner Bolte Patentanwälte
Rechtsanwälte Partnerschaft mbB
Hollerallee 73
28209 Bremen (DE)**

(56) References cited:
EP-A2- 0 897 755        WO-A1-2010/029994
WO-A1-2010/129994   WO-A1-2012/063374
WO-A1-2012/096378   WO-A1-2012/114076
JP-A- 2008 104 966      US-A- 4 753 579

• HARTMANN, C. S. ET AL.: 'Overview of design challenges for single phase unidirectional SAW filters' INVITED PAPER, 1989 IEEE ULTRASONICS SYMPOSIUM. 1989, pages 79 - 89, XP055284754

**Description**

FIELD OF THE INVENTION

The present invention is generally directed to apparatus for atomising liquids, and in particular to apparatus using surface acoustic wave (SAW) generation to atomise liquid. While the present invention will be described with reference to its use in medical applications, it is to be appreciated that the invention is not restricted to this application, and that other applications are also envisaged.

BACKGROUND OF THE INVENTION

Inhalation therapy is used to deliver pharmaceutical or other substances into the lungs of patients. In order for inhalation therapy to be effective, the liquid containing the substance to be delivered needs to be atomised to produce liquid droplets of a size that will allow the droplets to be readily inhaled and deposited within the patient's lungs. The droplets need to be less than 5 $\mu$m for deep lung deposition, and be of a larger size if deposition is required higher in the airways.

Atomisation apparatus using surface acoustic wave (SAW) propagation to atomise liquid have been considered for use for inhalation therapy. This is because they can potentially provide designs that are portable and allow for greater control of the atomisation rate and droplet size. These apparatus typically include a piezoelectric substrate having an upper working surface, and an interdigital transducer located on the upper working surface. Application of RF power to the interdigital transducer results in the generation of surface acoustic waves in the upper working surface. Liquid to be atomised is supplied to the upper working surface. While the amplitude of the surface vibration of the SAW is only in the order of 1 nm, the SAW is typically driven with a frequency over 10MHz. This results in an acceleration as high as $10^7$m/s$^2$, leading to strong liquid actuation when the SAW meets the liquid supported on the working surface. The acceleration is sufficient to overcome the capillary-viscous stress within the liquid and to break up the capillary wave generated in the liquid into micron sized aerosols. This is what is known as SAW atomisation.

Japanese Publication No. 2008-104966 (Seiko Epson Corp) describes a SAW atomisation apparatus which uses a porous member in contact with the upper working surface of a SAW actuator to supply liquid to that upper working surface for atomisation. The porous member acts as a wick to draw liquid from a reservoir to the upper working surface.

International publication no. WO 2010/129904 (Monash University) describes a SAW atomisation apparatus which is particularly applicable for use in inhalation therapy. An EWC-SPUDT type interdigital transducer is used to focus the SAW generated in the upper working surface of the piezoelectric substrate to a focus point leading to increased atomisation efficiency. The liquid to be atomised is supplied directly to the working surface of the SAW actuator through a thin paper wick in contact with the working surface. The liquid supplied to the working surface forms a liquid meniscus at the edge of the paper wick from which atomisation can take place. WO2012/063374 and WO2010/129994 disclose apparatus for atomising liquid generating SAW and a liquid delivery arrangement including a porous member in contact with a peripherial edge of a piezoelectric substrate.

The Applicants has found that in atomisation apparatus using SAW generation, supplying fluid directly to the working surface can lead to an overall loss of energy limiting the operational energy efficiency of the apparatus. This is because liquid supplied to the working surface of the SAW actuator participates in the vibration of the working surface prior to breaking up into atomised liquid droplets. The liquid, prior to atomisation, therefore absorbs some of the energy associated with the generated SAW limiting the operational energy efficiency of the apparatus. Furthermore, results have shown that part of the SAW energy is absorbed by the first 1 to 3 mm of the wick at the interface of the wick with the liquid meniscus.

Because of the limitation on the operational energy efficiency of such atomisation apparatus, relatively high power, typically around 3 to 6 watts, therefore needs to be applied to the interdigital transducer of the SAW actuator to operate correctly. This can lead to high localised heating at the transducer which can contribute to premature failure of the apparatus.

It would be advantageous to provide an atomisation apparatus with improved operational energy efficiency compared with prior art designs.

It is also preferably advantageous to provide an atomisation apparatus having improved reliability compared with prior art designs.

With this in mind, the present invention provides an apparatus for atomising liquid, according to the disclosure of claim 1.

As previously noted, the applicants have found that liquid supplied directly to the working surface using a wick in contact with the working surface will also participate in the vibration of that working surface prior to atomisation resulting in a loss of energy, and thereby limiting the overall operational energy efficiency of the atomisation apparatus. This energy loss is avoided according to the present invention by not supplying the liquid to be atomised directly to the working surface using a wick in contact with that surface. The piezoelectric substrate typically has a planar configuration with an upper working surface and peripheral edge surfaces extending along the periphery of the piezoelectric surface. The peripheral edge surface therefore extends at an angle, typically around 90 degrees, relatively to the plane of the working surface. The peripheral edge of the piezoelectric substrate is located where the peripheral edge surface meets the upper working surface of the piezoelectric substrate. The interaction of the SAW at the peripheral edge with the liquid being supplied by the porous member leads to formation of a thin liquid layer having a relatively large meniscus area from which atomisation of the liquid can occur. The SAW excitation at the peripheral edge of the piezoelectric substrate results in liquid being drawn from the porous member onto the working surface. Limiting the contact area of the porous member to the peripheral edge of the piezoelectric substrate limits the SAW energy being absorbed by the porous member helping to minimise energy loss from the apparatus.

The Applicants have studied the fluid flow mechanisms that help to drive liquid motions within SAW atomisation apparatus. It is the Applicant's understanding that the dominant mechanism driving liquid motion within a thin boundary layer adjacent to the working surface is "Schlichting' streaming (or boundary layer streaming). This mechanism drives liquid motion within the thin boundary layer through viscous shear which results in a net liquid motion towards the source of the SAW irradiation. This results in liquid being drawn from the porous member to form a liquid layer over the working surface adjacent the peripheral edge, the liquid layer having a liquid meniscus from which atomisation can take place.

According to the invention the thickness of the liquid layer formed over the working surface adjacent the peripheral edge is close to, or the same as the thickness of the boundary layer produced immediately above the working surface where the liquid directly interacts with the SAW vibration. This therefore allows the Schlichting streaming mechanism to dominate the the driving of the liquid within the liquid layer. The advantage provided by this is that it limits or prevents the appearance of what is known as "Eckart" streaming within the meniscus. Eckart streaming acts to drive flow in the bulk of the liquid in the direction of the SAW generation, and therefore in opposition to the Schlichting streaming. This can result in the production of aerosols of a large and therefore unsatisfactory size, for example greater than 100 $\mu$m in diameter.

The thickness of the boundary layer will be generally the same as the wavelength of the SAW propagating through the liquid. This wavelength may be determined using the following equation:

$$\lambda_f = {c_f}\!/\!{f} = 50 \mu m$$

where

$\lambda_f$ is the wavelength of the SAW generated in the working surface

$c_f$ is the travelling velocity of the SAW in water which is 1482 m/s; and

$f$ is the frequency of operation which is for example 30 MHz.

Therefore the boundary layer may have a thickness of about 50 $\mu$m, and the maximum thickness of the liquid layer is then preferably equal to or less than 50 $\mu$m. This is difficult to produce using a wick in direct contact with the working surface as in prior art SAW atomisation apparatus as the wick needs to be limited in thickness to 50 $\mu$m or less to enable a liquid layer of that thickness to be produced. This may be achieved, according to a preferred aspect of the invention, by positioning the porous member relative to the peripheral edge such that an upper surface of the porous member is spaced from the working surface at a distance generally corresponding to the desired thickness of the liquid layer. The liquid is then drawn from the portion of the porous member extending above the peripheral edge to thereby form a liquid layer on the working surface having the desired thickness. It is therefore not necessary to limit the thickness of the porous member as would be required where a porous wick was in contact with the working surface.

The porous member may preferably be formed from porous cellulose material which provides for controlled delivery of liquid. The porous member may for example be made from a biodegradable hydroxypropyl cellulose (HPC). The porous member may be shaped to thereby allow one end of the porous member may be in contact with at least one point on said peripheral edge of the piezoelectric substrate. Alternatively, the porous member may contact a length of the peripheral edge. The SAW may be focused at the part of the peripheral edge contacted by the porous member. The shape and pore size within the porous member can act to control the flow rate of liquid to the peripheral edge. The opposing end of the porous member may be supported within a container containing the liquid to be atomised. The end of the porous member within the liquid container may be located within a support element formed from absorbent material. This support element, in addition to supporting the porous member, also assists in transferring liquid from the container to the porous member.

Interdigital transducers (IDT) typically include a positive electrode and a negative electrode. Electrode fingers respectively extend from the positive and negative electrodes, the electrode fingers being located in an interlaced relationship on the working surface of the piezoelectric substrate. The IDT of the apparatus according to the present invention may preferably be a single phase, unidirectional transducer (SPUDT). The use of SPUDTs provides advantages over traditional IDTs. In a SAW actuator using a traditional IDT, the SAW propagates in both forward and reverse directions in the working surface. This is because the SAW is reflected back to the IDT from the edge of the SAW actuator. These reflections interfere with the forward moving SAW leading to a considerable loss in SAW energy. By comparison, the SAW generated from a SPUDT propagates in a single direction only. This is achieved by superimposing internally tuned reflector fingers between each pair of electrode fingers. These reflector fingers may be formed as electrode fingers extending from at least one of the electrodes. The placement of these reflector fingers enhances the SAW in the forward direction, and suppresses the SAW in the reverse direction. Therefore, less SAW energy is lost using a SPUDT, and the risk of interfering reflections is reduced.

Such SPUDTs can also be designed to focus the energy of the generated SAW to a predetermined area on the working surface by curving the fingers, preferably along an elliptical curve. The SPUDT may therefore focus the SAW to an area immediately adjacent a said peripheral edge surface of the piezoelectric substrate. The focus area may preferably be located immediately adjacent where the porous member is in contact with the peripheral edge surface. Alternatively, the SAW may be focused along a line aligned with the peripheral edge surface contacting the porous member.

The SPUDT may preferably be of a DART-SPUDT type. A DART-SPUDT has several advantages over other known SPUDTs, including the EWC-SPUDT electrode described in the previously mentioned International Publication No. WO 2010/299904. In SPUDTs, the width of each electrode finger, including the reflector fingers, is a function of the wavelength (A) generated by the electrode. In a DART-SPUDT, the reflector fingers is 3/8 A in width, while the reflector fingers of a EWC-SPUDT is ¼ A in width. Also, the reflector fingers of a Hanma-SPUDT are 3/16 λ in width. The reflector fingers of an EWC-SPUDT and a Hanma-SPUDT are therefore more narrow than the reflector fingers of a DART-SPUDT. The advantage of having an electrode with relatively wider reflector finger width is that this reduces the effect of electrode resistance and Joule heating thereby reducing the power required to be applied to the electrodes, and reducing localised heating of the transducer. Furthermore, the cost of producing the interdigital transducers on the piezoelectric substrate using manufacturing processes such as photolithography is significantly reduced when compared with interdigital transducers having narrower electrode fingers. In addition, the number of SAW actuators that need to be rejected due to defective interdigital transducers is significantly reduced.

It has also been identified by the Applicant that the thickness of the metal layer forming the IDT performs an important role in reducing power loss. It has been found that IDTs having a thickness of at least 1% the SAW wavelength, and preferably between 1 to 5% of the SAW wavelength are more efficient in delivering energy to the piezoelectric substrate. Normal IDTs have a thickness significantly less than this. Therefore, using the above mentioned equation, the IDT thickness may be between 1.32 and 6.6 $\mu$m, where the travelling velocity of the SAW is 3965 m/s in the piezoelectric substrate and the frequency of operation of the SAW actuator is 30MHz. There are a number of advantages provided by the additional thickness of the IDT. Firstly, it reduces the resistance, and consequently, the impedance of the IDT thereby improving the efficiency of the SAW actuator. This is particularly critical where the atomisation apparatus is battery powered. Another advantage is that the temperature of the SAW actuator is significantly reduced, typically from around 80°C to about 50°C. This is particularly important where biological and chemical carrying liquids, as such liquids are not able to handle high temperatures. For example, stem cells are unable to handle high temperatures, but are able to be delivered using the atomisation apparatus according to the present invention. The reduced operational temperature also improves the reliability of operation of the atomisation apparatus. This is because localised heating at the IDT is a direct contributor to premature failure of the device. In addition, the greater thickness of the IDT is useful in the case of SPUDT type transducers as it is useful in assisting in blocking the SAW reflections travelling in the reverse direction.

In prior art atomisation apparatus using SAW actuation, the power required to achieve atomisation is around 3 to 6 Watt, with the input voltage being as high as 60 V peak to peak (-20 $V_{RMS}$). By comparison, the present invention can preferably achieve atomisation with a greatly reduced power of 0.5-3 Watts, with the input voltage also at a much lower and safer range at only around 30 Volts peak-to-peak (-10 $V_{RMS}$). This is in large part because of the thickness of the IDT used that reduces the resistance, and consequently the impedance of the SAW actuator improving the efficiency of the apparatus as previously explained. The lower power and voltage requirements are important for consumer devices to assist in compliance with current safety regulations. It has been found that atomisation rate of over 200 $\mu$l/min can be achieved with the above mention power and voltage in an apparatus according to the present invention. By comparison, prior art apparatus need as much as 20 $V_{RMS}$ to achieve the same rate.

It should be noted that terms such as "upper" and "side" used to describe the relative positions of features of the present invention and therefore relate to one specific orientation of the atomisation apparatus. As the atomisation apparatus can also be used in alternative orientations, the use of the above-noted terms are not intended to limit the scope of the invention to being in that specific orientation.

The lower operational temperature of the atomisation apparatus according to the present invention, and therefore, the lower temperature of the aerosols/droplets produced allows for the delivery of biological substances such as the stem cells previously referred to which would otherwise be damaged if exposed to the higher operational temperatures of less efficient prior art apparatus. The atomisation apparatus may however also be used in other applications such as the atomisation of liquids containing fragrant substances, and chemicals and pharmaceuticals such as antibiotics for delivery into a single or multiple rooms of a building. Other potential uses for the apparatus include the delivery of fragrant substances such as perfumes and scents for both personal and room applications. It is also possible for the apparatus to be used to deliver cosmetic substances, prays for the mouth and throat of a person, sprays with cleaning, sterilizing, and anti-allergy substances, or agricultural chemicals such as herbicides, fungicides, insecticides and fertilizers. Other applications of the atomisation apparatus according to the present invention are also envisaged.

BRIEF DESCRIPTION OF THE DRAWINGS

It will be convenient to further describe the invention with reference to the accompanying drawings which illustrate a preferred embodiment of the present invention. Other embodiments are possible and, consequently, the particularity of the accompanying drawings is not to be understood as superseding the generality of the preceding description of the invention.

In the drawings:

Figure 1 is a schematic top view of an atomisation apparatus according to the present invention;

Figure 2 respectively show a plan view and detailed cross-sectional view of an interdigital electrode of the atomisation apparatus of Figure 1; and

Figure 3 is a detailed side view of the atomisation apparatus of Figure 1 showing the liquid delivery arrangement of the apparatus of Figure 1; and

Figure 4 is a detailed schematic view of the contact area of the peripheral edge of the piezoelectric substrate and the porous member of the atomisation apparatus of Figure 1.

DETAILED DESCRIPTION OF THE INVENTION

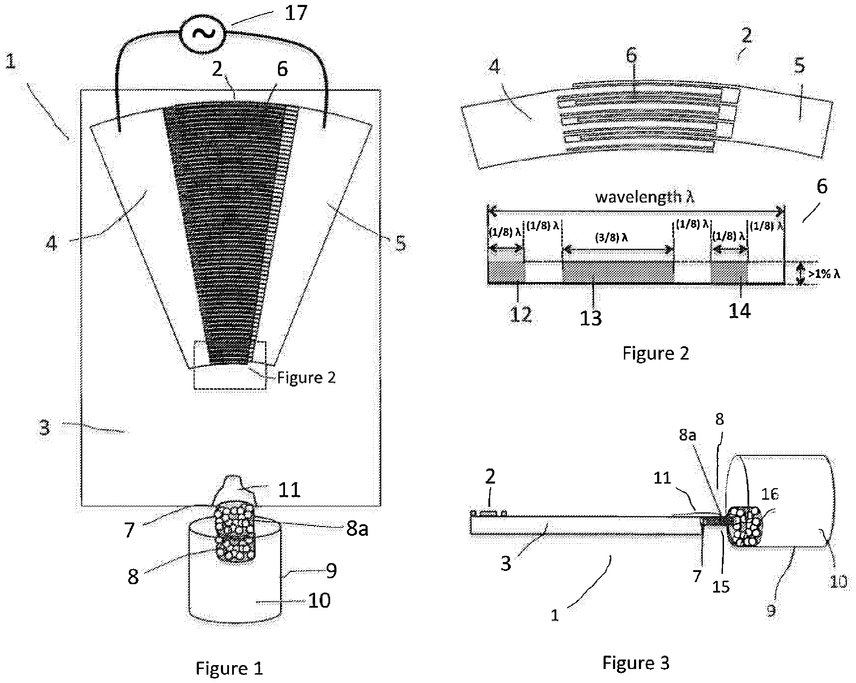

Referring initially to Figure 1, the SAW atomisation apparatus according to the present invention includes a piezoelectric substrate 1 which is typically formed from lithium niobate ($LiNbO_3$). Unlike more commonly used piezoelectric material such as lead zironate titanate (PZT), $LiNbO_3$ is lead free and therefore safe to use in medical applications. The piezoelectric substrate 1 has an upper working surface 3 through which an SAW can be generated. A peripheral edge 7 extends along the outer periphery of the piezoelectric substrate 1.

An interdigital transducer, preferably of a DART-SPUDT type, is located on the working surface 3. The interdigital transducer 2 includes a positive electrode 5 and a negative electrode 4, with electrode fingers 6 respectively extending from the positive and negative electrodes. The electrode fingers 6 of each electrode 4,5 are located in an interlaced relationship. Application of an electrical signal to the transducer element 2 results in the generation of an SAW through the working surface 3 of the piezoelectric substrate 1.

The liquid 10 to be atomised is accommodated within a liquid container 9. A porous member 8 extends from the liquid container 9, with one end of the porous member being in contact with the peripheral edge 7 of the piezoelectric substrate 1. The other end of the porous member 8 is supported by an absorbent support element 16 located within the liquid container 9. The support element 16 (as shown in Figure 3), as well as supporting the porous member 8, also facilitates the transfer of the liquid 10 from the container 9 through into the porous member 8. The porous member may for example be made from a polymer cellulose such as biodegradable hydroxypropyl cellulose (HPC).. The use of other hydrophilic materials for the porous member 8 is also envisaged.

Referring now to Figure 2 which provides a detailed view of the interdigital transducer 2, the electrode fingers 6 may have a elliptical or circular curved configuration. This arrangement allows the energy of the generated SAW to be directed to a predetermined area on the peripheral edge 7. In the SAW atomisation apparatus according to the present invention, the SAW can be directed to an area immediately adjacent to where the porous member 8 contacts the peripheral edge 7. It is also envisaged that the electrode fingers 6 be straight, in which case the generated SAW may be directed along a line extending parallel to the peripheral edge 7 contacting the porous member 8.

Extending from the negative electrode 4 are reflector fingers 13 that are wider than the other electrode fingers 12, 14. The purpose of the reflector fingers 13 is to prevent the reflections of the SAW in a reverse direction to the SAW propagating from the DART-SPUDT transducer 2, thereby minimising the loss of energy from the generated SAW. A DART-SPUDT transducer 2 differs from other SPUDT transducers, for example an EWC-SPUDT or Hanma-SPUDT type, in that the reflector fingers 13 have a width of 3/8 A as shown in Figure 2, A being the wavelength of the generated SAW. By comparison, an EWC-SPUDT has reflector fingers with a width of ¼ λ, and a Hanma-SPUDT has reflector fingers with a width of 3/16 A. DART -SPUDT transducers therefore have the widest reflector fingers of these SPUDT types. The use of larger SPUDTs has the benefit of reducing the effects of electrode resistance and Joule heating. Another advantage of having larger sized interdigital transducers is that it is easier to deposit the transducer 2 on the piezoelectric substrate 1 using photolithographic techniques. The transducer 2 can therefore be deposited with greater accuracy leading to a lower defect rate for these transducers.

Figure 2 shows in more detail the configuration of a DART-SPUDT transducer. The positive electrode 5 has an electrode finger 14 with a width of 1/8 λ, the positive electrode finger 14 being located and aligned with a negative electrode finger 12, and a reflector finger 13 extending from the negative electrode 4. The positive and negative electrode fingers 12, 14 and the reflector finger 13 are respectively spaced apart a distance of 1/8 λ.

As also shown in Figure 2, the thickness of the interdigital transducer 2 may be greater than 1 % λ, λ being the SAW wavelength. It is preferred that the thickness of the interdigital transducer 2 be between 1 to 5% λ. The transducer 2 therefore has a thickness that is greater than the thickness of traditional interdigital transducers. The greater thickness of the electrode fingers 6 results in lower relative impedance through the transducer 2 thereby improving the efficiency of the SAW atomisation apparatus. The improved efficiency means that a relatively lower amount of power need be applied to the transducer 2 to operate correctly. This furthermore reduces the local temperature of the aerosols that leave the apparatus to around 50°C.

The SAW generated within the working surface 3 moves in a general direction towards the peripheral edge 7 in contact with the porous member 8. When the SAW reaches the peripheral edge of the working surface 3, a physical phenomenon known as Schlichting streaming acts to draw liquid 10 from the porous member 8. The result of this Schlichting streaming phenomenon is that the liquid will try to move towards the source of the SAW. This results in an accumulation of liquid, in a thin liquid layer 11 adjacent the peripheral edge 7. This thin liquid layer 11 can extend across the working surface 3 from the peripheral edge 7. The thin liquid layer 11 may also extend over onto the upper surface 8a of the porous member 8 as shown in Fig. 3. This liquid volume forms a liquid meniscus 11a from which is generated the atomised liquid droplets.

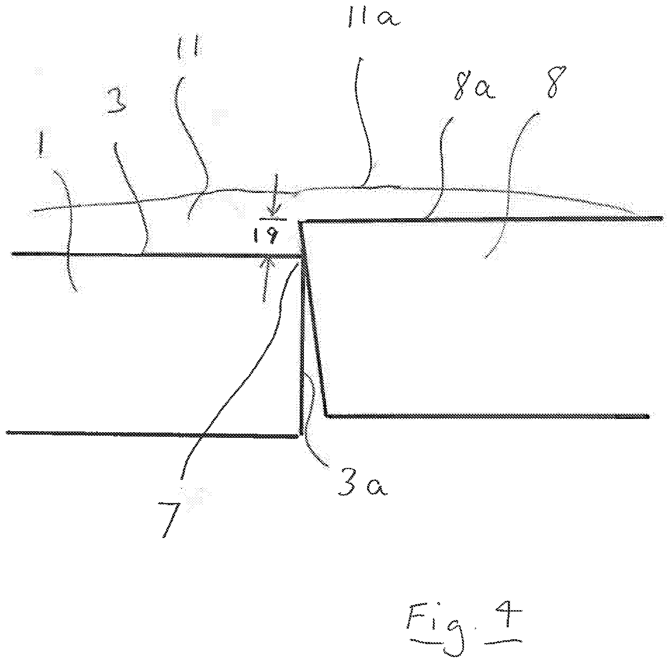

Figure 4 shows in more detail the configuration of the atomisation apparatus where the porous member 8 contacts the peripheral edge 7 of the piezoelectric substrate 1. The peripheral edge 7 is located where the working surface 3 meets a side surface 3a of the piezoelectric substrate 1. Figure 4, only shows the porous member 8 contacting the piezoelectric substrate 1 at the peripheral edge 7. It is however also possible for the porous member 8 to contact the side surface 3a of the piezoelectric substrate 1. The porous member 8 is located relative to the peripheral edge 7 such that the upper surface 8a of the porous member 8 is spaced a fixed distance 19 from the working surface 3. This distance 19 is preferably equal to or less than the boundary layer thickness which is generally equal to the wavelength of the SAW propagating though the working surface 3. This helps to ensure that the maximum thickness of the liquid layer 11 extending from the peripheral edge 7 is similar to the boundary layer thickness. This will help to ensure that Schlichting streaming dominates the liquid motion so that the liquid can be drawn from the porous member 8 to form the liquid layer 11, and that the liquid can subsequently be atomised to an optimal size from the meniscus 11a of that liquid layer 11..

As only a relatively small amount of liquid is drawn at any one time over the working surface, there is little involvement of that liquid volume with the SAW vibration generated in the working surface 3 leading to a reduced loss of energy from the atomisation apparatus when compared with other known apparatus. Furthermore, the limited contact of the porous member with the piezoelectric substrate limits any loss of SAW energy through being absorbed by the porous member. This results in improved operational energy efficiency for the atomisation apparatus according to the present invention. In addition, the reduced temperature of the aerosols/droplets produced by the atomisation apparatus according to the present invention allows for the delivery of more temperature sensitive biological substances, chemicals or pharmaceuticals.

## Claims

1. An apparatus for atomising liquid, including a piezoelectric substrate (1) having a working surface (3), and a peripheral edge (7) extending along a side of the working surface (3), an interdigital transducer (2) located on the working surface (3) for generating surface acoustic waves (SAW) in the working surface, and a liquid delivery arrangement including a porous member (8) for supplying the liquid (10) to be atomised, wherein the porous member is in contact with the peripheral edge (7) of the piezoelectric substrate (1); and **characterized in that** the porous member (8) is positioned relative to the peripheral edge such that an upper surface (8a) of the porous member (8) is spaced a distance (19) from the working surface (3) equal to or less than a thickness of a boundary layer located immediately adjacent the working surface within the liquid drawn from the porous member (8) by the SAW.

2. An apparatus for atomising liquid according to claim 1, wherein the interdigital transducer (2) is a DART-SPUDT.

3. An apparatus for atomising liquid according to any one of the preceding claims, wherein the interdigital transducer (2) has a thickness of at least 1 % of the SAW wavelength.

4. An apparatus for atomising liquid according to claim 3, wherein the thickness of the interdigital transducer (2) is between 1 to 5% of the SAW wavelength.

5. A method of delivery of fragrant substances using an apparatus according to any one of claims 1 to 4.

6. A method of delivering cosmetic substances using an apparatus according to any one of claims 1 to 4.

7. A method of generating sprays with cleaning, sterilizing, anti-allergy substances using an apparatus according to any one of claims 1 to 4.

8. A method of delivering agricultural chemicals including herbicides, fungicides, insecticides and fertilizers using an apparatus according to any one of claims 1 to 4.

**Patentansprüche**

1. Vorrichtung zur Atomisierung von Flüssigkeit, einschließlich einem piezoelektrischen Substrat (1) mit einer Arbeitsoberfläche (3) sowie einem peripheren Rand (7), der sich entlang einer Seite der Arbeitsoberfläche (3) erstreckt, einem Interdigitalwandler (2), der sich auf der Arbeitsoberfläche (3) befindet, zum Generieren von akustischen Oberwellen (SAW) in der Arbeitsoberfläche sowie einer Flüssigkeitsabgabeanordnung, die ein poröses Element (8) zum Zuführen der zu atomisierenden Flüssigkeit (10) einschließt, wobei das poröse Element in Kontakt mit dem peripheren Rand (7) des piezoelektrische Substrats (1) ist; und **dadurch gekennzeichnet, dass** das poröse Element (8) relativ zu dem peripheren Rand so positioniert ist, dass eine obere Oberfläche (8a) des porösen Elements (8) in einem Abstand (19) von der Arbeitsoberfläche (3) beabstandet ist, der gleich oder geringer als eine Dicke einer Grenzschicht ist, die sich unmittelbar benachbart zu der Arbeitsoberfläche innerhalb der Flüssigkeit befindet, die durch die SAW aus dem porösen Element (8) gezogen wird.

2. Vorrichtung zum Atomisieren von Flüssigkeit nach Anspruch 1, wobei der Interdigitalwandler (2) ein DART-SPUDT ist.

3. Vorrichtung zum Atomisieren von Flüssigkeit nach einem der vorhergehenden Ansprüche, wobei der Interdigitalwandler (2) eine Dicke von mindestens 1 % der Wellenlänge der SAW hat.

4. Vorrichtung zum Atomisieren von Flüssigkeit nach Anspruch 3, wobei die Dicke des Interdigitalwandlers (2) zwischen 1 und 5 % der Wellenlänge der SAW beträgt.

5. Verfahren zur Abgabe von Duftsubstanzen unter Verwendung einer Vorrichtung nach einem der Ansprüche 1 bis 4.

6. Verfahren zur Abgabe von Kosmetiksubstanzen unter Verwendung einer Vorrichtung nach einem der Ansprüche 1 bis 4.

7. Verfahren zum Generieren von Sprays mit reinigenden, sterilisierenden, antiallergischen Substanzen unter Verwendung einer Vorrichtung nach einem der Ansprüche 1 bis 4.

8. Verfahren zur Abgabe von Agrochemikalien einschließlich Herbiziden, Fungiziden, Insektiziden und Düngemitteln unter Verwendung einer Vorrichtung nach einem der Ansprüche 1 bis 4.

**Revendications**

1. Appareil destiné à atomiser un liquide, comportant un substrat piézoélectrique (1) ayant une surface de travail (3) et un bord périphérique (7) s'étendant le long d'un côté de la surface de travail (3), un transducteur interdigital (2) situé sur la surface de travail (3) destiné à générer des ondes acoustiques de surface (SAW) dans la surface de travail, et un agencement de délivrance de liquide comportant un élément poreux (8) destiné à fournir le liquide (10) à atomiser, l'élément poreux étant en contact avec le bord périphérique (7) du substrat piézoélectrique (1) ; et **caractérisé en ce que** l'élément poreux (8) est positionné par rapport au bord périphérique de telle sorte qu'une surface supérieure (8a) de l'élément poreux (8) est espacée de la surface de travail (3) par une distance (19) égale ou inférieure à une épaisseur d'une couche limite située de façon immédiatement adjacente à la surface de travail à l'intérieur du liquide extrait de l'élément poreux (8) par les SAW.

2. Appareil destiné à atomiser un liquide selon la revendication 1, dans lequel le transducteur interdigital (2) est un DART-SPUDT.

3. Appareil destiné à atomiser un liquide selon l'une quelconque des revendications précédentes, dans lequel le transducteur interdigital (2) a une épaisseur représentant au moins 1 % de la longueur d'onde des SAW.

4. Appareil destiné à atomiser un liquide selon la revendication 3, dans lequel l'épaisseur du transducteur interdigital (2) représente entre 1 et 5 % de la longueur d'onde des SAW.

5. Procédé de délivrance de substances parfumées au moyen d'un appareil selon l'une quelconque des revendications 1 à 4.

6. Procédé de délivrance de substances cosmétiques au moyen d'un appareil selon l'une quelconque des revendications 1 à 4.

7. Procédé de production d'aérosols avec des substances nettoyantes, stérilisantes, antiallergiques au moyen d'un appareil selon l'une quelconque des revendications 1 à 4.

8. Procédé de délivrance de produits agrochimiques, y compris d'herbicides, de fongicides, d'insecticides et de fertilisants, au moyen d'un appareil selon l'une quelconque des revendications 1 à 4.

Figure 1

wavelength λ

(1/8) λ   (1/8) λ   (3/8) λ   (1/8) λ   (1/8) λ   (1/8) λ

>1% λ

12     13     14

Figure 2

Figure 3

EP 2 961 454 B1

Fig. 4

**REFERENCES CITED IN THE DESCRIPTION**

**Patent documents cited in the description**

- JP 2008104966 A
- WO 2010129904 A
- WO 2012063374 A
- WO 2010129994 A
- WO 2010299904 A